# EUROPEAN PATENT APPLICATION

(11) **EP 4 721 650 A1**
(43) Date of publication of application: **08.04.2026**
(21) Application number: 24819448.2
(22) Date of filing: 06.03.2024
(51) Int. Cl.: A61B 5/02, A61B 5/026, A61B 5/00, G16H 50/20, G16H 50/30, G16H 30/20, G16H 10/60

(54) **METHOD AND DEVICE FOR EARLY DIAGNOSIS AND MANAGEMENT OF VARICOSE VEINS**

(30) Priority: 05.06.2023 KR 20230072047
(71) Applicant: EX Healthcare Inc., Seongnam-si, Gyeonggi-do 13449 (KR)
(72) Inventor: LEE, Yong Hee, Sujeong-gu Gyeonggi-do 13449 (KR); SHIN, Hong Ju, Sujeong-gu Gyeonggi-do 13449 (KR); CHOI, Hyangtae, Sujeong-gu Gyeonggi-do 13449 (KR)
(74) Representative: Reddie & Grose LLP
(86) International application number: PCT/KR2024/002872
(87) International publication number: WO 2024/253295

(57) **Abstract**

Examples relate to a method for early diagnosis and management of varicose veins by a server, comprising acquiring varicose veins-related information from a user device, digitizing a capillary exposure based on analysis of the varicose veins-related information, evaluating a risk of varicose veins based on the digitized capillary exposure, and transmitting information of varicose veins evaluation results based on the evaluated risk of varicose veins to the user device.

## Description

### [TECHNICAL FIELD]

Examples relate to a method and a device for managing varicose veins through early diagnosis of varicose veins. More specifically, they relate to a method and a device for early diagnosis and management of varicose veins by analyzing images acquired from a user device.

### [BACKGROUND ART]

Varicose veins may be a disease in which superficial veins of lower extremities are abnormally swollen due to dysfunction of one-way valve dysfunction of lower extremities. In the early stage, varicose veins may not have any special symptoms other than discomfort, but if it is left and progresses gradually, various kinds of complications may occur. In general, the incidence of varicose veins of lower extremities increases with aging, and it is a disease that occurs more in women than in men.

The number of patients with varicose veins tends to be increasing gradually, and the social cost according thereto is also increasing. Therefore, in order to reduce the disease of varicose veins and the social cost according thereto, it is needed to establish and manage an evaluation system for early diagnosis and management of varicose veins in advance. Considering the afore-mentioned points, a method for early diagnosis and management of varicose veins through a non-fact-to-fact service platform will be described below.

### [PRIOR ARTS]

### [PATENT DOCUMENTS]

(Patent document 1) Korean Patent Publication No. 10-2022-0019180A

### [DISCLOSURE]

### [TECHNICAL PROBLEM]

The present description can provide a method for early diagnosis and management of varicose veins.

The present description can provide a method for a platform that acquires image information from a user device for early diagnosis of varicose veins, and evaluates a risk through this.

The present description can provide a method for early diagnosis and management of varicose veins through a non-face-to-face service platform.

The present description can provide a method for extracting an image for determining varicose veins.

The present description can provide a method for determining a risk of varicose veins by analyzing a varicose veins-related image.

Problems to be solved by the present description are not limited to those described above, and can be expanded to various matters that can be derived by the examples of the invention described below.

### [TECHNICAL SOLUTION]

According to one example of the present description, a method for early diagnosis and management of varicose veins by a server, may comprise acquiring varicose veins-related information from a user device, digitizing a capillary exposure based on analysis of the varicose veins-related information, evaluating a risk of varicose veins based on the digitized capillary exposure, and transmitting information of varicose veins evaluation results based on the evaluated risk of varicose veins to the user device.

In addition, according to one example of the present description, a server for early diagnosis and management of varicose veins, may comprises a memory storing varicose veins-related information, a transceiver performing communication with a user device, and a processor controlling the memory and the transceiver, wherein the processor, may acquire varicose veins-related information from the user device, and a capillary exposure may be digitized based on analysis of the varicose veins-related information, and a risk of varicose veins may be evaluated based on the digitized capillary exposure, and information of the varicose veins evaluation results may be transmitted to the user device based on the evaluated risk of varicose veins.

Furthermore, according to one example of the present description, a method for early diagnosis and management of varicose veins by a user device, may comprise transmitting varicose veins-related information to a server, and acquiring information of varicose veins evaluation results from the server based on a risk of varicose veins, wherein the server may digitize a capillary exposure based on analysis of the varicose veins-related information, and evaluate the risk of varicose veins based on the digitized capillary exposure to generate the information of varicose veins evaluation results.

In addition, the following matters may be applied in common.

According to one example of the present description, the varicose veins-related information may comprise at least any one of varicose veins-related images, varicose veins-related videos, and additional images.

In addition, according to one example of the present description, the varicose veins-related images may be images of a lower body part of a user, and when a capillary exposure is digitized based on analysis of the varicose veins-related information, the server may derive parameters by pixel unit for the lower body part of the user from the varicose veins-related images, and identify capillaries by deriving a standard deviation based on a plurality of the parameters by pixel unit, and digitize the capillary exposure through a capillary with a preset value or higher among the identified capillaries.

In addition, according to one example of the present description, when the risk of varicose veins is evaluated on the basis of the digitized capillary exposure, a plurality of capillary lines may be identified, and the risk of varicose veins may be evaluated by weighting the degree of capillary distribution of a preset value or higher in a plurality of the capillary lines, and when a number of capillary with the preset value or higher are distributed in any one capillary line of a plurality of the capillary lines, the risk of varicose veins may increase.

Furthermore, according to one example of the present description, the server may acquire a plurality of varicose veins-related images, and a plurality of the varicose veins-related images may be images for the same lower body part of the user, and the capillary exposure may be digitized and compared from each of a plurality of the varicose veins-related images, and the risk of varicose veins may be evaluated by weighting the information of the comparison results.

In addition, according to one example of the present description, a first image of a plurality of the varicose veins-related images may be an image acquired in a first time zone, and a second image may be an image acquired in a second time zone, and the information of the comparison results may be acquired by comparing the capillary exposure of the first image and the capillary exposure of the second image, and the greater the difference between the capillary exposure of the first image and the capillary exposure of the second image based on the information of the comparison results, the risk of varicose veins may increase.

Moreover, according to one example of the present description, the server may collect user information of user activities between the first time zone and the second time zone through at least any one of the user device and a wearable device, and the collected user information may be reflected to evaluation of the risk of varicose veins.

In addition, according to one example of the present description, the varicose veins-related video may be a video of a lower body part of a user taken vertically, and the server may derive a plurality of varicose veins-related images from the varicose veins-related video.

Furthermore, according to one example of the present description, the server may provide guide information for photographing the varicose veins-related video to the user device, and when the varicose veins-related video photographed by the user device based on the guide information is transmitted to the server, the varicose veins-related video may be encrypted and transmitted.

In addition, according to one example of the present description, the server may acquire user questionnaire information from the user device, and the risk of varicose veins may be evaluated by reflecting the user questionnaire information.

### [ADVANTAGEOUS EFFECTS]

The present description has an effect of providing a method for early diagnosis and management of varicose veins.

The present description has an effect of providing a method for a platform that acquires image information from a user device for early diagnosis of varicose veins, and evaluates a risk through this.

The present description has an effect of providing a method for early diagnosis and management of varicose veins through a non-face-to-face service platform.

The present description has an effect of providing a method for extracting an image for determining varicose veins.

The present description has an effect of providing a method for determining a risk of varicose veins by analyzing a varicose veins-related image.

Problems to be solved by the present description are not limited to those described above, and can be expanded to various matters that can be derived by the examples of the invention described below.

### [BRIEF DESCRIPTION OF THE DRAWINGS]

FIG. 1 is a drawing that illustrates an example of the operation environment of the system according to one example of the present description.
FIG. 2 is a block diagram for describing the internal configuration of the computing device (200) in one example of the present description.
FIG. 3a is a drawing that shows the service platform and server operation for early diagnosis and management of varicose veins in one example of the present invention.
FIG. 3b is a drawing that shows the service platform and server operation for early diagnosis and management of varicose veins in one example of the present invention.
FIG. 3c is a drawing that shows the method for providing information of a risk of varicose veins to a user through the service platform for early diagnosis and management of varicose veins in one example of the present description.
FIG. 4 is a drawing that shows the operations of the user device and server in one example of the present description.
FIG. 5 is a drawing that shows the method for deriving an image from a video for diagnosing varicose veins, in one example of the present description.
FIG. 6 is a drawing that shows an image related to a risk of varicose veins in one example of the present description.
FIG. 7 is a drawing that shows an image related to a risk of varicose veins in one example of the present description.
FIG. 8a is a drawing that shows the method for utilizing a plurality of images for diagnosis of varicose veins in one example of the present description.
FIG. 8b is a drawing that shows the method for utilizing a plurality of images for diagnosis of varicose veins in one example of the present description.
FIG. 9 is a drawing that shows the method for analyzing a varicose veins-related image for evaluation of a risk according to one example of the present description.
FIG. 10 is a drawing that shows the method for analyzing a varicose veins-related image for evaluation of a risk according to one example of the present description.
FIG. 11 is a drawing that shows the method for analyzing a varicose veins-related image for evaluation of a risk according to one example of the present description.
FIG. 12 is a flowchart that shows the method for early diagnosis and management of varicose veins according to one example of the present description.

### [MODE FOR INVENTION]

In describing the examples of the present description, when it is judged that specific description of a known configuration or function may obscure the gist of the examples of the present description, detailed description thereof will be omitted. In addition, parts which are not related to the description of the examples of the present description in the drawings have been omitted, and similar reference numerals have been given for similar parts.

In the examples of the present description, when any component is said to be "connected", "coupled", or "accessed" to another component, this may include not only a direct connection relation, but also an indirect connection relation in which another component is present therebetween. In addition, when any component is said to "comprise" or "has" another component, unless otherwise mentioned particularly, this does not exclude another component, but this means to further comprise another component.

In the examples of the present description, terms such as first, second, and the like are used only for the purpose of distinguishing one component from another component, and unless otherwise mentioned particularly, they do not limit the order or importance or the like of components. Accordingly, within the scope of the examples of the present description, a first component may be referred to as a second component, and in the same manner, a second component in an example may be referred to as a first component in another example.

In the examples of the present description, components that are distinguished from each other are intended to clearly describe each characteristic, and do not mean that the components are necessarily separated. In other words, a plurality of components may be integrated to form one hardware or software unit, and one component may be distributed to form a plurality of hardware or software units. Therefore, even if not mentioned separately, such integrated or distributed examples are included in the scope of the examples of the present description.

In the present description, a network may be a concept that includes both wired and wireless networks. Then, the network may mean a communication network in which data exchange may be performed between a device and a system and between devices, and is not limited to a specific network.

The examples described in the present description may have aspects that are entirely hardware, or partially hardware, and are partially software, or entirely software. In the present description, "unit", "device" or "system" or the like refers to a computer-related entity such as hardware, a combination of hardware and software, or software, or the like. For example, in the present description, unit, module, device, or system, or the like may be a process, a processor, an object, an executable, a thread of execution, a program, and/or a computer that are running, but not limited thereto. For example, both an application running on a computer and a computer may correspond to the unit, module, device or system, or the like of the present description.

In addition, in the present description, the device may be a mobile device such as a smart phone, a tablet PC, a wearable device, and an HMD (Head Mounted Display), as well as a fixed device such as PC or a home appliance with a display function. Furthermore, as one example, the device may be an in-vehicle cluster or IoT (Internet of Things). In other words, in the present description, the device may refer to devices capable of operating an application, and is not limited to a specific type. In the following, for convenience of explanation, a device on which an application operates refers to as a device.

In the present description, the communication method of the network is not limited, and the connection between each component may not be connected by the same network method. The network may include not only a communication method that utilizes a communication network (as one example, a mobile communication network, wired Internet, wireless Internet, a broadcasting network, a satellite network, etc.), but also short-range wireless communication between devices. For example, the network, may include all communication methods by which an object and ab object can network, and is not limited to wired communication, wireless communication, 3G, 4G, 5G, or other methods. For example, the wired and/or network may refer to a communication network by one or more communication methods selected from the group consisting of LAN (Local Area Network), MAN (Metropolitan Area Network), GSM (Global System for Mobile Network), EDGE (Enhanced Data GSM Environment), HSDPA (High Speed Downlink Packet Access), W-CDMA (Wideband Code Division Multiple Access), CDMA (Code Division Multiple Access), TDMA (Time Division Multiple Access), Bluetooth, Zigbee, Wi-Fi, VoIP (Voice over Internet Protocol), LTE Advanced, IEEE802.16m, WirelessMAN-Advanced, HSPA+, 3GPP Long Term Evolution (LTE), Mobile WiMAX (IEEE 802.16e), UMB (formerly EV-DO Rev. C), Flash-OFDM, iBurst and MBWA (IEEE 802.20) systems, HIPERMAN, Beam-Division Multiple Access (BDMA), Wi-MAX (World Interoperability for Microwave Access) and ultrasonic utilization communication, but not limited thereto.

The components described in various examples do not necessarily mean essential components, and some may be optional components. Accordingly, examples that are composed of a subset of the components described in the examples may be also included in the scope of the examples of the present description. In addition, examples comprising other components additionally to the components described in various examples are also included in the scope of the examples of the present description.

Hereinafter, the examples of the present description will be described in detail with reference to the drawings.

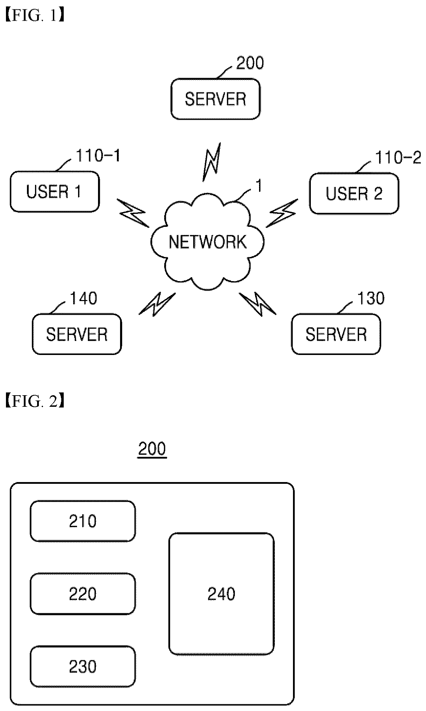

FIG. 1 is a drawing that illustrates an example of the operation environment of the system according to one example of the present description.

Referring to FIG. 1, one or more user devices (110-1, 110-2), and one or more servers (120, 130, 140) are connected through a network (1). This FIG. 1 is one example for explanation of the invention, and the number of the user devices and the number of the servers are not limited as in FIG. 1.

One or more user devices (110-1, 110-2) may be a fixed terminal or a mobile terminal implemented by a computer system. The one or more user devices (110-1, 110-2) include, for example, smart phones, mobile phones, navigation devices, computers, laptops, digital broadcasting terminals, PDA (Personal Digital Assistants), PMP (Portable Multimedia Player), tablet PCs, game consoles, wearable devices, IoT (internet of things) devices, VR (virtual reality) devices, AR (augmented reality) devices, and the like. As one example, in the examples, the user device (110) may substantially mean one of various physical computer systems that can communicate with other servers (120 - 140) through the network (1) using a wireless or wired communication method.

Each server may be implemented as a computer device or a plurality of computer devices which communicate with the one or more user devices through the network (1) to provide instructions, codes, files, contents, services, and the like. For example, the server may be a system providing each service to one or more user devices (110-1, 110-2) accessed through the network (1). As a more specific example, the server may be a computer program installed and operated on the one or more user devices (110-1, 110-2), and may provide a service (as one example, information provision, etc.) intended by the corresponding application to the one or more user devices (110-1, 110-2). As another example, the server may distribute a file for installation and operation of the afore-mentioned application to the one or more user devices (110-1, 110-2) and receive user input information to provide a corresponding service.

FIG. 2 is a block diagram for describing the internal configuration of the computing device (200) in one example of the present description. This computing device (200) may be applied to the afore-mentioned one or more user devices (110-1, 110-2) or servers (120-140) referring to FIG. 1, and each of the devices and servers may have the same or a similar internal configuration as being composed by adding or excluding some components.

Referring to FIG. 2, the computing device (200) may comprise a memory (210), a processor (220), a communication module (230), and a transceiver (240). The memory (210) is a non-transitory computer readable recording medium, and may include a permanent mass storage device such as an RAM (random access memory), an ROM (read only memory), a disk drive, an SSD (solid state drive), a flash memory, and the like. Herein, the permanent mass storage device such as the ROM, SSD, flash memory, disk drive, and the like is a separate permanent storage device that is distinguished from the memory (210), and may be comprised in the afore-mentioned device or server. In addition, in the memory (210), an operating system and at least one program code (as one example, a browser installed on the user device (110) and the like, or a code for an application and the like installed in the user device (110) and the like for providing a specific service) may be stored. Such software components may be loaded from a separate computer readable recording medium from the memory (210). Such a separate computer readable recording medium may include computer readable recording media such as a floppy drive, a disk, a tape, a DVD/CD-ROM drive, a memory card, and the like.

In other example, the software components may be loaded in the memory (210) through a communication module (230), not a computer readable recording medium. For example, at least one program may be loaded in the memory (210) based on a computer program (as one example, the afore-mentioned application) installed by files which developers or a file distribution system (as one example, the afore-mentioned server) that distributes an installation file of an application provides through the network (1).

The processor (220) may be configured to process instructions of a computer program, by performing basic arithmetic, logic and input and output arithmetic operations. The instructions may be provided to the processor (220) by the memory (210) or communication module (230). For example, the processor (220) may be configured to execute instructions received according to a program code stored in a recording device such as the memory (210).

The communication module (230) may provide a function for the user device (110) and the server (120 -140) to communicate with each other through the network (1), and may provide a function for each of the device (110) and/or server (120 - 140) to communicate with other electronic devices.

The transceiver (240) may be a means for interfacing with an external input/output device (not shown). For example, the external input device may include a device such as a keyboard, a mouse, a microphone, a camera, and the like, and the external output device may include a device such as a display, a speaker, a haptic feedback device, and the like. As other example, the transceiver (240) may be a means for interfacing with a device in which functions for input and output are integrated into one such as a touch screen.

In addition, in other examples, the computing device (200) may comprise much more components than the components of FIG. 2 depending on the properties of the applied device. For example, when the computing device (200) is applied to the user device (100), it may be implemented to comprise at least some of the afore-mentioned input and output devices, or further comprise other components such as a transceiver, a GPS (Global Positioning System) module, a camera, various kinds of sensors, a database, and the like. As a more specific example, when the user device is a smartphone, it may be implemented to further comprise various components such as an acceleration sensor or a gyro sensor, a camera module, various kinds of physical buttons, buttons using a touch panel, input and output ports, and a vibrator for vibration and the like which are generally comprised in a smartphone.

In the following, it will be described based on the computing device (200) operating based on FIG. 1 and FIG. 2. As one example, a service platform for early diagnosis of varicose veins may be provided based on at least any one of applications, software and programs operated in the computing device (200).

Herein, the service platform for early diagnosis of varicose veins may be installed and operated in the user device, and the corresponding platform may be controlled by the server. The server may provide a service by exchanging data with the user device as the computing device (200) through the network. As one example, a program or software for determining a risk of varicose veins by acquiring an image or video and analyzing it through the service platform for early diagnosis of varicose veins may be constructed in the server. In addition, the server can construct an artificial intelligence model or use an external artificial intelligence model to increase the accuracy of determination of the risk of varicose veins, which will be described later.

As other one example, the service platform for early diagnosis of varicose veins may be a platform managed based on a plurality of nodes, and is not limited to a specific form. In other words, the service platform for early diagnosis of varicose veins may be managed through the server or other nodes, and provide a service to a user through an application and other program operated by the computing device (200), and may not be limited to a specific form. As one example, the service platform for early diagnosis of varicose veins may be provided in various forms to a user, but in the following, for convenience of explanation, the service platform for early diagnosis of varicose veins will be described based on a platform operated in the user device (300). However, this is merely one example for convenience of explanation, but may be not limited thereto.

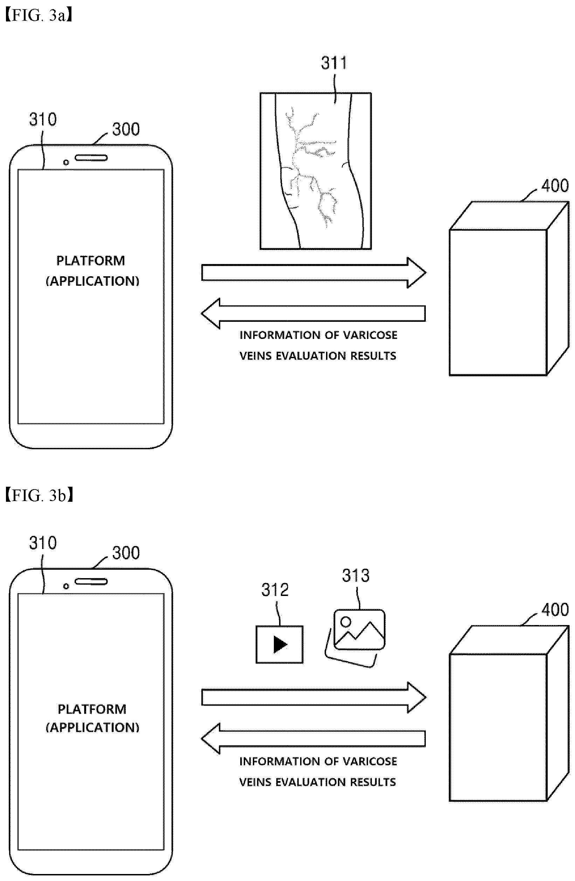

FIG. 3a to FIG. 3c are drawings that show the service platform and server operation for early diagnosis and management of varicose veins in one example of the present invention. Referring to FIG. 3a, the service platform (or application) for early diagnosis of varicose veins (hereinafter, service platform, 310) may be operated in the user device (300). As one example, the user device (300) may transmit a varicose veins-related image (311) to the server (400) through the service platform (310), and the server (400) may provide information of varicose veins evaluation results that evaluates a risk of varicose veins through the acquired varicose veins-related image (311) to the service platform (310) operated in the user device (300). In other words, a user may be provided with a service for determining a risk of varicose veins through the service platform (310) installed in the user device (300). Herein, as one example, the risk of varicose veins may be determined by the varicose veins-related image (311). More specifically, varicose veins may be a disease in which superficial veins of lower extremities are abnormally swollen due to dysfunction of one-way valve dysfunction of lower extremities, and most of symptoms of the corresponding disease may be concentrated in the calf or thigh area. Herein, when symptoms are severe, capillaries in the calf or thigh area may swell to a level at which they can be confirmed with naked eyes. In other words, the capillary exposure of the calf or thigh may be a measure for diagnosing varicose veins or determining a risk. As one example, for determining a risk of varicose veins, the capillary exposure may be digitized.

In addition, as a criterion for determining varicose veins, the degree of edema may be further used in addition to the capillary exposure. The degree of edema may mean the degree of volume or volume change of a specific area. Moreover, for the degree of edema, the volume or volume change of the calf or instep may be a target of judgement, but the present invention is not limited thereto.

Considering the afore-mentioned, the risk of varicose veins may be judged based on the digitized capillary exposure in the varicose veins-related image (311). In other words, early diagnosis of varicose veins may be performed by determining how much of capillaries are exposed and expressed in the thigh or calf image transmitted by the user to derive the digitized capillary exposure, and based on this, determining the risk of varicose veins. The server (400) may acquire the image of varicose veins (311) related to the user from the service platform (310) operated in the user device (300), and determine the risk of varicose veins therefrom, and then provide the corresponding information through the service platform (310).

Herein, as one example, to increase the accuracy of judgement of the risk of varicose veins, the analysis accuracy for the image of varicose veins (311) may be increased or a number of images of varicose veins (311) may be needed. As one specific example, the user status may be changed in real time. In addition, a specific area of the user may show severe symptoms, while other specific area may show mild symptoms. In other words, since there may be limitations in accurately expressing the user status by the images of varicose veins (311) acquired by the server (400), to accurately determining the user status, a number of images may be required. In addition, since the judgement of the risk of varicose veins is not performed through face-to-face medical treatment, and is performed by a non-face-to-face image, limitations may exist. Accordingly, in order to accurately determine the risk of the images of varicose veins (311), a method for increasing the image analysis accuracy may be required.

Herein, as one example, referring to FIG. 3b, the server (400) may further acquire not only the images of varicose veins (311) but also varicose veins videos (312) and varicose veins-related additional images (313). In other words, the server (400) may perform analysis of the risk of varicose veins through the videos (312) or related additional images (313), and this will be described later.

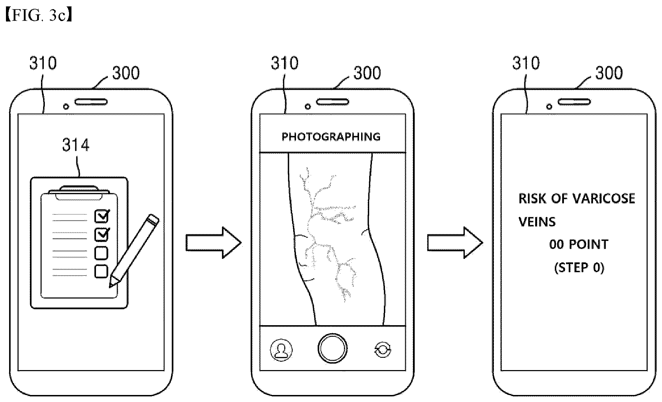

Furthermore, as one example, referring to FIG. 3c, the server (400) may further acquire user questionnaire information (314) from the service platform (310), and by reflecting this, the risk of varicose veins may be determined. Specifically, when the user intends to be provided with a service for judgement of the risk of varicose veins through the service platform (310) operated in the user device (300), the user may input user questionnaire information (314) to provide it to the server (400) and then acquire a video or image to provide it to the server (400). The server (400) may determine the risk of varicose veins through the user questionnaire information (314) and video or image information acquired through the service platform (310) of the user device (300), and provide information of varicose veins risk evaluation results to the user through the service platform (310). Herein, the information of the risk of varicose veins is digitized information, and may be expressed as a risk score or risk level, but may not be limited to a specific form.

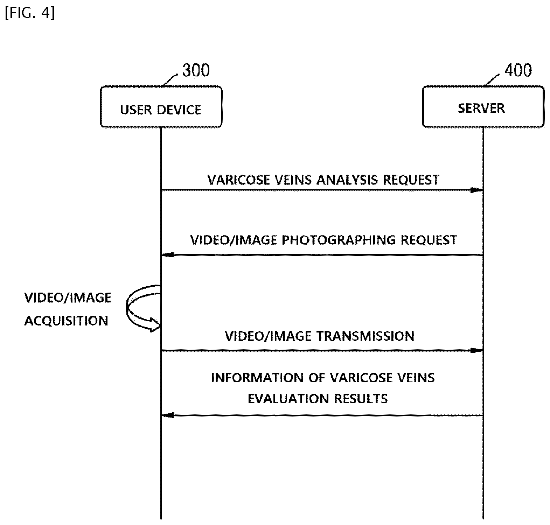

Specifically, FIG. 4 is a drawing that shows the operations of the user device and server in one example of the present description. Referring to FIG. 4, the user device (300) may transmit a request for analysis of varicose veins to the server (400). Herein, the request for analysis of varicose veins may be transmitted with the user questionnaire information (314) to the server (400). In other words, the user may transmit the request for analysis of varicose veins together with the user questionnaire information to the server (400) through the service platform (310) operated in the user device (300). After that, the server (400) may request photographing at least one of videos and images to the user device (300) for judgement of the risk of varicose veins. Herein, as one example, a plurality of the videos and images may be requested at regular time intervals, and this will be described later. The server (400) may acquire at least one of videos and images from the user device (300), and determine the risk of varicose veins based on this, and thereby, the information of the varicose veins evaluation results may be transmitted to the user device (300).

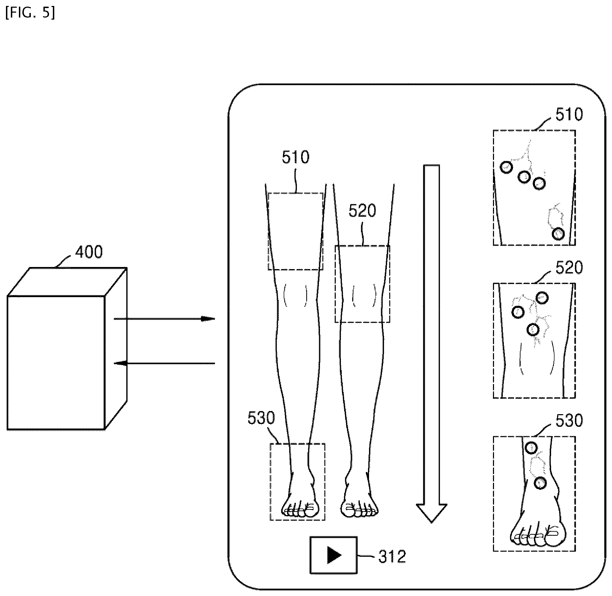

As one example, FIG. 5 is a drawing that shows the method for deriving an image from a video for diagnosing varicose veins, in one example of the present description. Referring to FIG. 5, the server may request the video (312) to the user device (300) and acquire it in order to increase the accuracy of evaluation of the risk of varicose veins. Herein, as one example, the video (312) may be a video of a lower body part of a human body, which is a video of a lower body taken vertically. As one specific example, the video (312) may be an image of the entire lower body, which is a video taking all of the left, right, front and back. In addition, as one example, it may be a video taking inside of the lower body, and is not limited to a specific form. Herein, photography guide information may be provided through the service platform (310), and the user may acquire a video by photographing a lower body part through the photography guide of the service platform (310). As other one example, since the afore-mentioned lower body part may be personal body information, the video may be encrypted and transmitted to the server (400) through the service platform (310). In other words, in the server (400), the corresponding video may be encrypted so that it cannot be played, and may be provided only by input of a program, software or artificial intelligence for determining the risk of varicose veins. The server (400) may extract varicose veins-related images (510, 520, 530) from the acquired video (312). In other words, at least one image (510, 520, 530) which is related to varicose veins by confirming the degree of swelling of capillaries in the video (312) may be extracted. Herein, as one example, the server (400) may be connected to the artificial intelligence (500), and the video (312) may be provided by input of the artificial intelligence (500) so that the afore-mentioned images (510, 520, 530) can be derived as output. As one example, the artificial intelligence (500) may be equipped with a learning model related to varicose veins, and be learned based on a plurality of images related to varicose veins, capillary digitization information and information on the risk of varicose veins. In other words, the artificial intelligence (500) may establish a learning model related to diagnosis of varicose veins, and through this, extract varicose veins-related images. After that, the server (400) may analyze the corresponding images to evaluate the risk of varicose veins. In addition, the artificial intelligence (500) may delete unnecessary information for determining the risk of varicose veins from the varicose veins-related images. As one example, the artificial intelligence (500) may delete information unrelated to determination of the risk of varicose veins on leg hair or others from the varicose veins-related images, and the corresponding information may be also recognized by learning. In other words, the artificial intelligence (500) may not only derive an image for determining the risk of varicose veins, but also delete unnecessary parts from the image, and increase the accuracy of determining the risk through the varicose veins-related image in which unnecessary information is deleted.

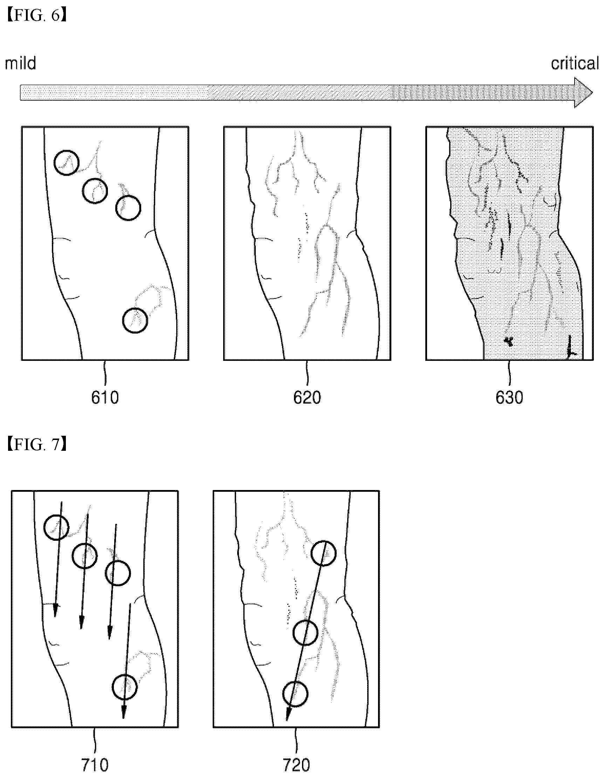

Herein, as one example, FIG. 6 and FIG. 7 are drawings that show an image related to a risk of varicose veins in one example of the present description. Referring to FIG. 6, the risk of varicose veins may be determined based on the images of varicose veins (610, 620, 630). As one example, the first image (610) may have only some capillaries exposed in the corresponding area, but the capillary exposure may not be high and not be in a swollen shape. In other words, the first image (610) may have a small capillary exposure numerical value. On the other hand, the second image (620) may have a higher capillary exposure than the first image, and the third image (630) may have the highest capillary exposure numerical value. In other words, the risk of varicose veins may be determined according to the capillary exposure numerical value, and it is needed to derive the capillary exposure numerical value, which is the degree to which capillaries are exposed in each image (610, 620, 630).

In addition, referring to FIG. 7, the risk of varicose veins may be evaluated in consideration of distribution of the exposed area of capillaries. Herein, the first image (710) may be a case where capillaries are present multiply in a plurality of capillary lines, and the second image (720) may be a case where many capillaries are exposed to one capillary line. Herein, considering symptoms of varicose veins, when many capillaries are exposed to one capillary line, it may be judged that varicose veins are further progressed. Therefore, the risk of the second image (720) may be higher than that of the first image (710). As one example, when analysis on the varicose veins-related image is performed, digitization through the exposed area of capillaries in the varicose veins-related image may be performed. Herein, considering each position of the exposed area of capillaries, whether the exposed area of capillaries is positioned in the same capillary line may be reflected in evaluation of the risk. In other words, the afore-mentioned information may be weighted, and in the corresponding case, the risk of varicose veins may be set to be shown higher. As one example, in FIG. 7, the risk may be set to be higher in the second image (720) than the first image (710).

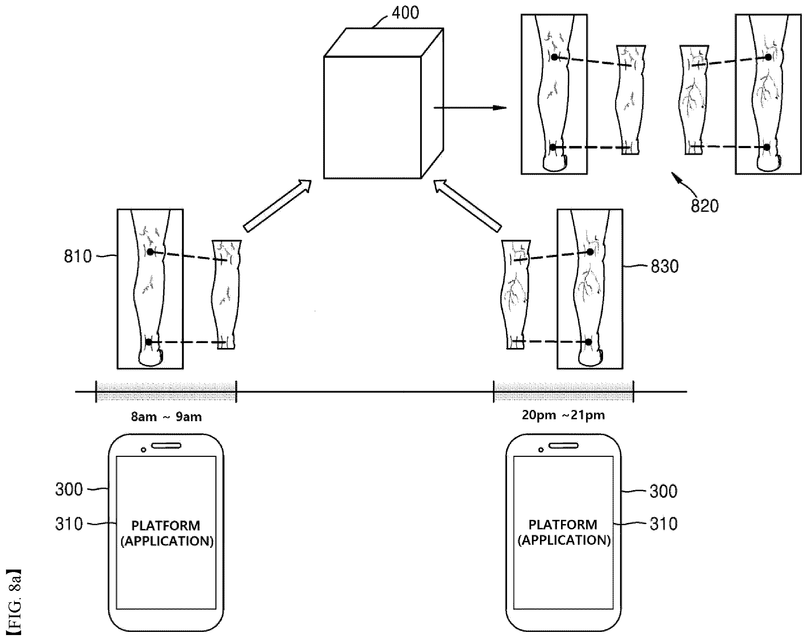

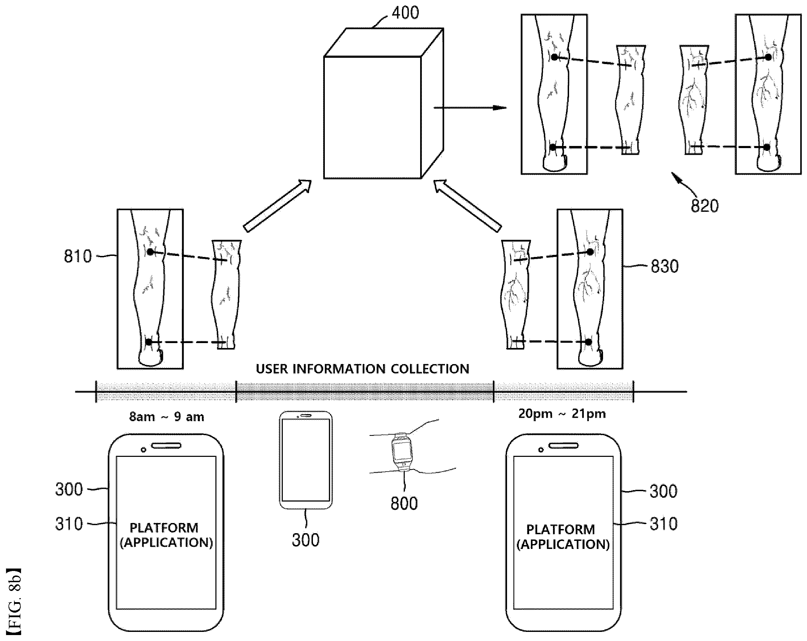

As another example, FIG. 8a and FIG. 8b are drawings that show the method for utilizing a plurality of images for diagnosis of varicose veins in one example of the present description. Referring to FIG. 8a, the server (400) may acquire a plurality of at least any one of the varicose veins-related images (311) and videos (312). It will be described based on the varicose veins-related images (311) for convenience of description below, and it may be applied in the same manner to the videos (312). In other words, the server (400) may analyze a plurality of varicose veins-related images (311) to increase the accuracy of analysis of varicose veins. Herein, as one example, in relation to symptoms of varicose veins, leg edema may be most evident in the evening after a user performs daily activities. Considering the afore-mentioned points, the server (400) may analyze the risk of varicose veins by acquiring a plurality of varicose veins-related images (311).

Herein, as one specific example, the server (400) may acquire the varicose veins-related images (311) based on different time zones. As one example, since the exposed areas of capillaries in the morning and evening time zones are different, the server (400) may acquire the varicose veins-related images (311) in each time zone. The server (400) may acquire information on the capillary exposure compared to a plurality of varicose veins-related images (311) and reflect them to the risk. As one example, the degree of capillary exposure may increase by the user's activities or other actions in relation to symptoms of varicose veins. In other words, the higher the degree of progression of varicose veins, the more remarkable the symptoms may be due to the user's activities or actions. Considering the above, the greater the changes in the image in the morning time zone and the image in the evening time zone in the varicose veins-related images (311) for the same area, the higher the risk of varicose veins may be. The server (400) may weight to the difference by analyzing a plurality of the images of varicose veins (311) acquired in the different time zones, and set the risk to be higher as the difference is greater. Herein, as one example, referring to FIG. 8b, user information may be collected between the time of acquiring the varicose veins-related images (311), and the corresponding information may be reflected to evaluate the risk. As one example, the service platform (310) may be driven in the user device (300) and the user's wearable device (800). Herein, the service platform (310) may collect user information between the time sections in which the images of varicose veins (311) are acquired, and acquire information related to the user' activities and actions. As one example, when the user's activity amount is low between the time of acquiring the images of varicose veins (311), the difference in the changes in the capillary exposure may be small. On the other hand, the difference in the changes in the capillary exposure may be large, when the user's activity amount is large, such as mountain climbing or other activities between the acquisition of the images of varicose veins (311). In other words, the user's activity amount needs to be considered when determining the risk of varicose veins, and for this, the server (400) may acquire user collection information and reflect the weight. The server (400) may determine the risk of varicose veins based on the user numerical information weight, and acquire user information through the user device (300) or wearable device (800) for the afore-mentioned operation.

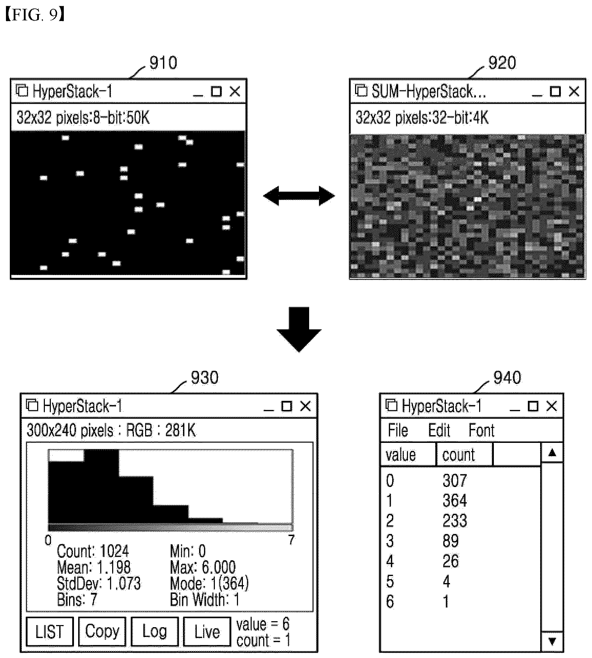

FIG. 9 is a drawing that shows the method for analyzing a varicose veins-related image for evaluation of a risk according to one example of the present description. Referring to FIG. 9, when the server (400) evaluates the risk of varicose veins through the varicose veins-related images (311), the server (400) may derive the human body part in the images (311) as parameters by pixel unit, and acquire standard deviation information for each pixel parameter value. Herein, information on the degree of distribution of capillaries may be identified based on each pixel parameter value and standard deviation, and the capillary exposure may be digitized. As one example, in FIG. 9, by reflecting the information (920) that calculates the standard deviation for each pixel, through information (910) on values above a certain level, information on the risk of varicose veins (930, 940) may be derived. In other words, it may be judged that capillaries are exposed in the pixels exceeding a preset value based on the standard deviation between skin pixels, and the capillary exposure may be digitized based on the number of the corresponding pixels, thereby determining the risk of varicose veins. Herein, as one example, whether multiple capillary exposures are present in a capillary line or whether multiple capillary exposures are reflected to multiple capillary lines as described above may be reflected on determination of the risk of varicose veins based on the afore-mentioned pixel information. In addition, the afore-mentioned information may be calculated for a plurality of the images of varicose veins, and information on the difference in digitized pixel changes in a plurality of images may be reflected to evaluation of the risk, as described above.

As one example, when a varicose veins-related image is acquired by the user device (300), it may not be easy to secure an image suitable for analysis. As one example, since the user may bend the waist or bend the legs when the user photographs the back of the calf, there may be limitations on acquisition of a plurality of images in the same posture, and correction for this may be needed. Considering the above, it is possible to further consider information related to the varicose veins-related images (311) and the user posture. As one example, the server (400) may acquire information on changes in the exposed part of capillaries by the user posture in advance, and based on this, the images may be corrected. In other words, the server (400) may perform correction on the images by further reflecting information of the user photographing posture in the acquired varicose veins-related images (311), and through this, the accuracy of evaluation of the risk may be increased. As one example, since the skin color may be reflected differently when the user posture is bent, the accuracy of determination of the risk may be increased by correcting the skin color based on the video taken in advance.

As other one example, for evaluation of the risk of varicose veins, the afore-mentioned artificial intelligence (500) may be utilized. Herein, the artificial intelligence (500) may be initially learned based on the doctor's clinical judgement information, and after that, may be learned based on reinforcement learning or other deep learning. In other words, for evaluation of the risk of varicose veins, the artificial intelligence (500) model may be constructed, and the artificial intelligence (500) may derive the risk evaluation value as output by reflecting the weight on the afore-mentioned risk and other learning information.

As other one example, user associated information may be provided based on determination of the risk of varicose veins of the server (400). As one example, the server (400) may provide a notification to a user whose value of evaluation of the risk of varicose veins is output high through the service platform (310). In addition, the server (400) may be associated with a hospital or other facility. The server (400) may perform confirmation of varicose veins by checking biomarkers through samples (e.g., saliva, urine, etc.) in a hospital or other facility for a user, and may enable to perform management of varicose veins for the corresponding user. As one example, the server (400) may provide a reservation system for a hospital specializing in varicose veins, a varicose veins check service, and other programs to a user through the service platform (310), and it may enable the user to manage varicose veins through the corresponding service platform (310).

As another one example, the server (400) may acquire additional information by linking with another device (e.g. human body component measuring device). As one example, the server (400) may further acquire lower body blood flow analysis information measured from another device and reflect it to evaluation of the risk of varicose veins, and is not limited to a specific example.

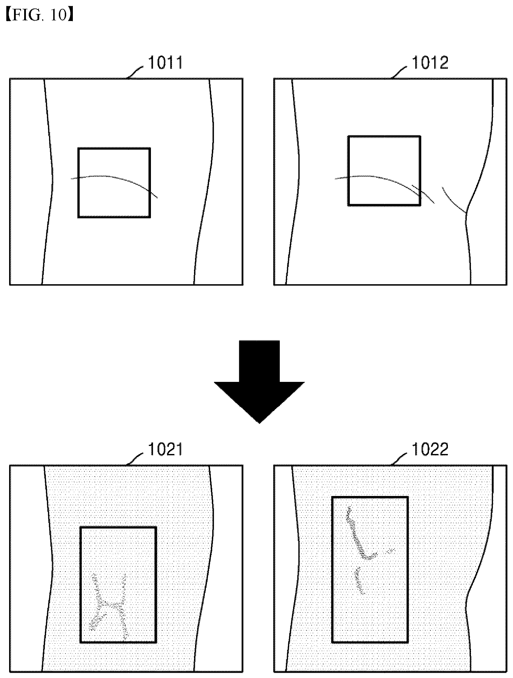

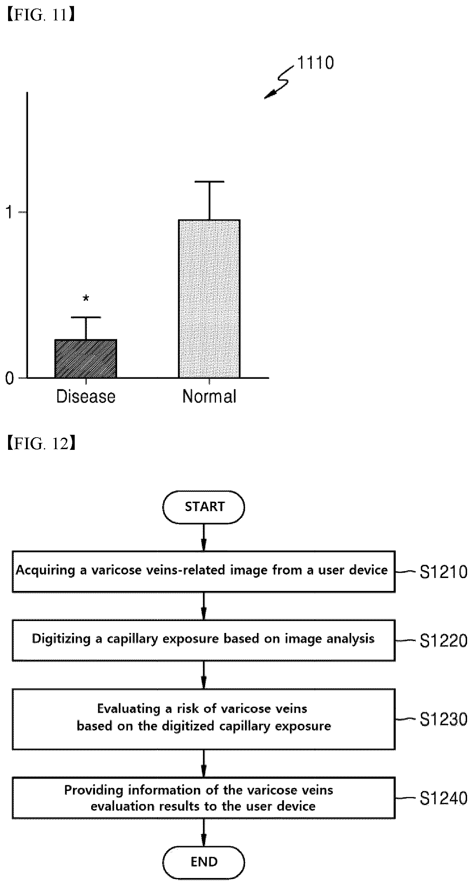

FIG. 10 and FIG. 11 are drawings that show the method for analyzing a varicose veins-related image for evaluation of a risk according to one example of the present description. Referring to FIG. 10, the server (400) may acquire varicose veins-related images (1011, 1012), acquire images (1021, 1022) as a result of analysis through the analysis described above, and evaluate the risk based on this. Herein, as described above, for evaluation of the risk, other information may be further utilized, but this is same as described above. Through this, a tool for early diagnosis of patients with varicose veins may be provided, and the therapeutic effect may be increased and the social costs may be reduced. As one example, since varicose veins are often left, when they are diagnosed at a hospital, the degree of progression may be already increased, and the therapeutic effect may be low. Therefore, through the afore-mentioned service platform (310) and server (400), the risk of varicose veins may be checked in a precautionary way before initial symptoms of varicose veins are expressed or symptoms are shown, and according to this, the cost and period of treatment may be reduced. In addition, through the afore-mentioned service platform (310) and server (400), non-face-to-face management of varicose veins may be performed without inconvenience of visiting a hospital. Furthermore, late diagnosis due to inconvenience of visiting a hospital even if initial symptoms are expressed may act as a factor which makes treatment difficult, makes the therapeutic effect lower, and increases treatment costs, and it is possible to perform a test simply without visiting a hospital through the afore-mentioned service platform (310) and server (400), and therefore, it may have a positive effect on preventing and treating varicose veins in daily life. In addition, as one example, discovery of diagnostic biomarkers and factors may be performed based on the data secured through the afore-mentioned service platform (310) and server (400), and FIG. 11 may be referred.

FIG. 12 is a flowchart that shows the method for early diagnosis and management of varicose veins according to one example of the present description.

Referring to FIG. 12, the server (400) may acquire varicose veins-related information from the user device (300). (S1210) After that, the server (400) may digitize the capillary exposure based on analysis of varicose veins-related information (S1220), and evaluate the risk of varicose veins based on the digitized capillary exposure. (S1230) Then, the server (400) may transmit information of varicose veins evaluation results to the user device (300) based on the evaluated risk of varicose veins. (S1240) Herein, the varicose veins-related information may comprise at least any one of varicose veins-related images, varicose veins-related videos, and additional images. As one example, the additional image may be an image of another body part of a user or another image, and is not limited to a specific form.

Furthermore, as one example, the varicose veins-related image may be an image of the user's lower body part. When the capillary exposure is digitized based on analysis of varicose veins-related information, the server may identify capillaries by deriving parameters by pixel unit for the user's lower body part, and deriving a standard deviation based on a plurality of parameters by pixel unit, from the varicose veins-related images, and digitize the capillary exposure through capillaries with a value higher than the preset value among the identified capillaries, and this is same as described above.

As other one example, when the risk of varicose veins is evaluated based on the digitized capillary exposure, the risk of varicose veins may be evaluated by identifying a plurality of capillary lines, and weighting the degree of distribution of capillaries with a value higher than the preset value in a plurality of the capillary lines. Herein, when many capillaries with a value higher than the preset value are distributed in any one capillary line of a plurality of the capillary lines, the risk of varicose veins may increase.

In addition, the server (400) may acquire a plurality of varicose veins-related images. Herein, a plurality of the varicose veins-related images may be images for the same lower body part of the user, and the capillary exposures may be digitized and compared in each of a plurality of the varicose veins-related images, and information of the comparison results may be weighted to evaluate the risk of varicose veins. As one specific examples, among a plurality of the varicose veins-related images, the first image may be an image acquired in the first time zone, and the second image may be an image acquired in the second time zone. The server (400) may acquire information of the comparison results by comparing the capillary exposure of the first image and the capillary exposure of the second image. Herein, based on the information of the comparison results, as the difference between the capillary exposure of the first image and the capillary exposure of the second image is greater, the risk of varicose veins may increase.

In addition, the server (400) may collect user information on the user activities between the first time zone and the second time zone through at least any one of the user device (300) and wearable device (800), and reflect the collected user information to evaluation of the risk of varicose veins.

As other one example, the varicose veins-related video may be a video of a lower body part of a user taken vertically. The server (400) may derive a plurality of varicose veins-related images from the varicose veins-related video. In addition, the server (400) may provide guide information for photographing a varicose veins-related video to the user device. Herein, when he varicose veins-related video taken by the user device based on the guide information is transmitted to the server, the varicose veins-related video may be encrypted and transmitted. In addition, the server (400) may acquire user questionnaire information from the user device (300), and reflect the user questionnaire information to evaluate the risk of varicose veins, and this is as described above.

The examples described above may be implemented at least partially as a computer program and be recorded on a computer readable recording medium. The computer readable recording medium in which a program to implement examples is recorded includes all kinds of recording devices in which data that can be readable by a computer are stored. Examples of the computer readable recording medium include ROM, RAM, CD-ROM, magnetic tape, optical data storage devices, and the like. In addition, the computer readable recoding medium may be distributed to a computer system connected with a network, so a computer readable code may be stored and implemented in a distribution manner. In addition, functional programs, codes, and code segments to implement the present examples may be easily understood by those skilled in the art to which the present examples pertain.

The present description examined above has been described with reference to the examples illustrated in the drawings, but they are merely exemplary, and those skilled in the art will understand that various modifications and modifications of examples are possible therefrom. However, such modifications should be considered to be within the technical protection scope of the present description. Thus, the true technical protection scope of the present description should be determined to include other embodiments, other examples, and equivalents to the claims by the technical spirit of the appended claims.

### [INDUSTIRAL AVAILABILITY]

The present description can provide a method for a platform that acquires image information from a user device for early diagnosis of varicose veins, and evaluates a risk through this, and thus, it is industrially available.

## Claims

1. A method for early diagnosis and management of varicose veins by a server, comprising acquiring varicose veins-related information from a user device;
digitizing a capillary exposure based on analysis of the varicose veins-related information; evaluating a risk of varicose veins based on the digitized capillary exposure; and
transmitting information of varicose veins evaluation results based on the evaluated risk of varicose veins to the user device.

2. The method according to claim 1,
wherein the varicose veins-related information comprises at least any one of varicose veins-related images, varicose veins-related videos, and additional images.

3. The method according to claim 2,
wherein the varicose veins-related images are images of a lower body part of a user, and
when a capillary exposure is digitized based on analysis of the varicose veins-related information, the server derives parameters by pixel unit for the lower body part of the user from the varicose veins-related images, and
identifies capillaries by deriving a standard deviation based on a plurality of the parameters by pixel unit, and
digitizes the capillary exposure through a capillary with a preset value or higher among the identified capillaries.

4. The method according to claim 3,
wherein when the risk of varicose veins is evaluated on the basis of the digitized capillary exposure, a plurality of capillary lines is identified, and the risk of varicose veins is evaluated by weighting the degree of capillary distribution of a preset value or higher in a plurality of the capillary lines, and
when a number of capillaries with the preset value or higher are distributed in any one capillary line of a plurality of the capillary lines, the risk of varicose veins increases.

5. The method according to claim 3,
wherein the server acquires a plurality of varicose veins-related images, and a plurality of the varicose veins-related images is images for the same lower body part of the user, and
the capillary exposure is digitized and compared from each of a plurality of the varicose veins-related images, and the risk of varicose veins is evaluated by weighting the information of the comparison results.

6. The method according to claim 5,
wherein a first image of a plurality of the varicose veins-related images is an image acquired in a first time zone, and a second image is an image acquired in a second time zone, and
the information of the comparison results is acquired by comparing the capillary exposure of the first image and the capillary exposure of the second image, and
the greater the difference between the capillary exposure of the first image and the capillary exposure of the second image based on the information of the comparison results, the risk of varicose veins may increase.

7. The method according to claim 6,
wherein the server collects user information of user activities between the first time zone and the second time zone through at least any one of the user device and a wearable device, and
the collected user information is reflected to evaluation of the risk of varicose veins.

8. The method according to claim 2,
wherein the varicose veins-related video is a video of a lower body part of a user taken vertically, and the server derives a plurality of varicose veins-related images from the varicose veins-related video.

9. The method according to claim 8,
wherein the server provides guide information for photographing the varicose veins-related video to the user device, and when the varicose veins-related video photographed by the user device based on the guide information is transmitted to the server, the varicose veins-related video is encrypted and transmitted.

10. The method according to claim 1,
wherein the server acquires user questionnaire information from the user device, and the risk of varicose veins is evaluated by reflecting the user questionnaire information.

11. A computer program stored in a computer readable medium which is combined with hardware to execute the method according to any one claim of claim 1 to claim 10.

12. A server for early diagnosis and management of varicose veins,
comprising a memory storing varicose veins-related information;
a transceiver performing communication with a user device; and
a processor controlling the memory and the transceiver,
wherein the processor,
acquires varicose veins-related information from the user device, and
a capillary exposure is digitized based on analysis of the varicose veins-related information, and a risk of varicose veins is evaluated based on the digitized capillary exposure, and
information of the varicose veins evaluation results is transmitted to the user device based on the evaluated risk of varicose veins.

13. A method for early diagnosis and management of varicose veins by a user device,
comprising transmitting varicose veins-related information to a server; and
acquiring information of varicose veins evaluation results from the server based on a risk of varicose veins,
wherein the server digitizes a capillary exposure based on analysis of the varicose veins-related information, and evaluates the risk of varicose veins based on the digitized capillary exposure to generate the information of varicose veins evaluation results.

14. The method according to claim 13,
wherein the varicose veins-related information comprises at least any one of varicose veins-related images, varicose veins-related videos, and additional images.

15. The method according to claim 14,
wherein the varicose veins-related images are images of a lower body part of a user, and
when a capillary exposure is digitized based on analysis of the varicose veins-related information, the server derives parameters by pixel unit for the lower body part of the user from the varicose veins-related images, and
identifies capillaries by deriving a standard deviation based on a plurality of the parameters by pixel unit, and
digitizes the capillary exposure through a capillary with a preset value or higher among the identified capillaries.

16. The method according to claim 15,
wherein when the risk of varicose veins is evaluated on the basis of the digitized capillary exposure, a plurality of capillary lines is confirmed, and the risk of varicose veins is evaluated by weighting the degree of capillary distribution of a preset value or higher in a plurality of the capillary lines, and
when a number of capillaries with the preset value or higher are distributed in any one capillary line of a plurality of the capillary lines, the risk of varicose veins increases.

17. The method according to claim 13,
wherein the user device acquires user questionnaire information and transmit it to the server, and the risk of varicose veins is evaluated by reflecting the user questionnaire information.
